Europäisches Patentamt

European Patent Office

Office européen des brevets

(19)

(11) **EP 1 659 402 A2**

(12) # EUROPEAN PATENT APPLICATION

(43) Date of publication:
**24.05.2006 Bulletin 2006/21**

(51) Int Cl.:
*G01N 33/36* (2006.01)    *G01N 19/08* (2006.01)

(21) Application number: 05466018.8

(22) Date of filing: **16.11.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **19.11.2004 CZ 20041131**

(71) Applicant: **Vyzkumny Ustav Textilnich Stroju Liberec a.s.**
**461 19 Liberec (CZ)**

(72) Inventors:
• **Kloucek, Pavel**
**460 10 Liberec (CZ)**

• **Sidlof, Petr.**
**460 14 Liberec (CZ)**
• **Hudousek, Ondrej**
**460 01 Liberec (CZ)**
• **Skop Petr.**
**460 01 Liberec (CZ)**
• **Sidlof, Pavel**
**460 04 Liberec (CZ)**
• **Cejka, Vaclav**
**460 13 Liberec (CZ)**

(74) Representative: **Musil, Dobroslav**
**Cejl 38**
**602 00 Brno (CZ)**

(54) **Method of measuring the longitudinal irregularities of slivers of textile fibres and of similar longitudinal formations and a device for carrying out the method**

(57) The invention relates to the measuring method of longitudinal irregularity of the sliver (1) of textile fibres and of a similar longitudinal formation, at which the sliver (1) or the similar longitudinal formations passes through the condensing funnel during the measuring, at the same time the length of the sliver (1) or of a similar longitudinal formation passed through the condensing funnel (2) is being measured and a value of forces by which the sliver (1) or a similar longitudinal formation is acting on the condensing funnel (2) and based on the course of the forces acting on the condensing funnel (2) the parameters of the mass irregularity of the sliver (1) or of a similar longitudinal formation are being determined.

The invention also relates to the device for measuring of mass irregularity of the sliver (1) of textile fibres and of a similar longitudinal formation that contains feeding, draw-off and measuring mechanism of the sliver (1) of textile fibres and of a similar longitudinal formation whose measuring mechanism contains the condensing funnel (2) of the sliver (1) and of a similar longitudinal formation that is positioned on the sensor (3) which is coupled with a device for processing the signal from the sensor (3) which is coupled with the sensing device of length of the sliver (1) and of a similar longitudinal formation.

**Obr. 1**

**Description**

**Technical field**

The invention relates to the method of measuring the longitudinal irregularities of slivers of textile fibres and of similar longitudinal formations.

A device for measuring the weight irregularity of the sliver of textile fibres and of a similar longitudinal formation containing the feeding, draw-off and measuring device of the sliver of textile fibres and of a similar longitudinal formation.

**Background** art

Production of textile yarns and of similar relatively thin and long textile formations is based on processing the slivers of textile fibres that have markedly larger cross section than the yarns or similar formations. The periodical and incidental mass irregularities of slivers are at their processing transformed into the products i.e. yarns at which after then both the strength as well as the diameter are varying. Especially the longitudinal periodical variation of yarn diameter of certain lengths or after certain intervals interferes the appearance of woven fabrics and knitted fabrics produced of such yarns and e.g. the moiré effect occurs. Weak spots at yarns and similar longitudinal formations then increase the probability of breakage due to which the productivity of woven fabrics and knitted fabrics production is being reduced. Therefore production of quality fibre slivers is very important, which can be achieved only through a frequent and immediate checking.

For measuring of longitudinal irregularities of slivers of textile fibres mostly the laboratory instruments are used for which the samples of slivers of necessary length are taken in operation from carding machines and drawing frames producing the sliver of textile fibres. These samples are transported into the air-conditioned laboratory where measuring on the respective instruments is being performed. The sliver is here fed between two opposite boards of the measuring condensator where the sliver together with the air creates a combined dielectric. During measurement a pair of draw-off rollers with the drive ensures that the sliver moves between the boards of instrument. At the beginning of measurement on several metres of sliver the mean value of length sliver weight is determined, after then during measuring of following metres of sliver the determined mean value is subtracted from values measured. Through this the percentage variations of longitudinal mass irregularity of sliver are received. The curve of measured variations of length weight is further analysed and the column spectrograms are determined, on basis of which the wave lengths of period irregularities of length weight are determined, e.g. for identification which one of the rotational parts of the machine causes the increased amplitude of columns in spectrograms. Next to this the longitudinal variation curve CV(L) for evaluation of carding process and for regulation of carding process and the percentage coefficient of quadratic irregularity CV is being determined. In a similar way, only upon usage of other measuring condensator, the measurement is being performed also on the produced yarn. The measured length of sliver and of yarn is being determined base on the speed of movement and time of measurement.

The disadvantage of the present state-of the art is the substantial required length of boards of measuring condensator and therefore the variations of length sliver weight cannot be measured, i.e. the longitudinal irregularity with the wave length shorter than the length of boards of the measuring condensator. Another disadvantage is not quite exact identification of wave length of periodical irregularities from the column spectrograms. The next disadvantage is also the necessity to determine the mean value of length sliver weight still before the measuring itself, through which the length of sliver brought into laboratory is consumed in vain still before each measuring. The next disadvantage is restriction of using the measuring method only to the laboratory space to which, moreover, the slivers and possibly the yarns must be brought from the production machines in an intact status. Finally, the last disadvantage of the present state of the art is the high acquisition cost of measuring instruments.

The goal of the invention is to eliminate or at least to minimise the disadvantages of present state of the art.

**The principle of invention**

The goal of the invention has been reached by measurement method of longitudinal irregularities of textile fibre slivers and of similar longitudinal formations whose principle consists in that the sliver or a similar longitudinal formation passes through the condensing funnel during the measuring, at the same time the length of sliver or of a similar longitudinal formation passed through the condensing funnel is being measured and the value of forces at which the sliver or a similar longitudinal formation is acting on the condensing funnel and based on the course of forces acting on the condensing funnel, the parameters of mass irregularity of sliver or of a similar longitudinal formation are determined.

This method is exact and reliable and may be used as an independent measuring device, or on the new production machines and also on the current production machines as an additional accessory.

To improve evaluation of measurement, it is advantageous if the rapid changes of length weight of sliver or of a similar longitudinal formation are filtered off during measuring.

The principle of the device for measuring the longitudinal irregularities of slivers of textile fibres and of similar longitudinal formations is that the measuring mechanism contains the condensing funnel of sliver and of a similar longitudinal formation which is positioned on the pressure transmitter that is coupled with the device for processing the signal from the sensor, at the same time this is coupled with sensing device of sliver length and of a similar longitudinal formation.

This device is reliable, durable, at the same time enables continuous and inexpensive upgrading. The device may be installed directly on the production machine, both on the new machine or the used one.

The suitable executions of the device as per the invention are shown in the related patent claims to the device.

## Description of the drawing

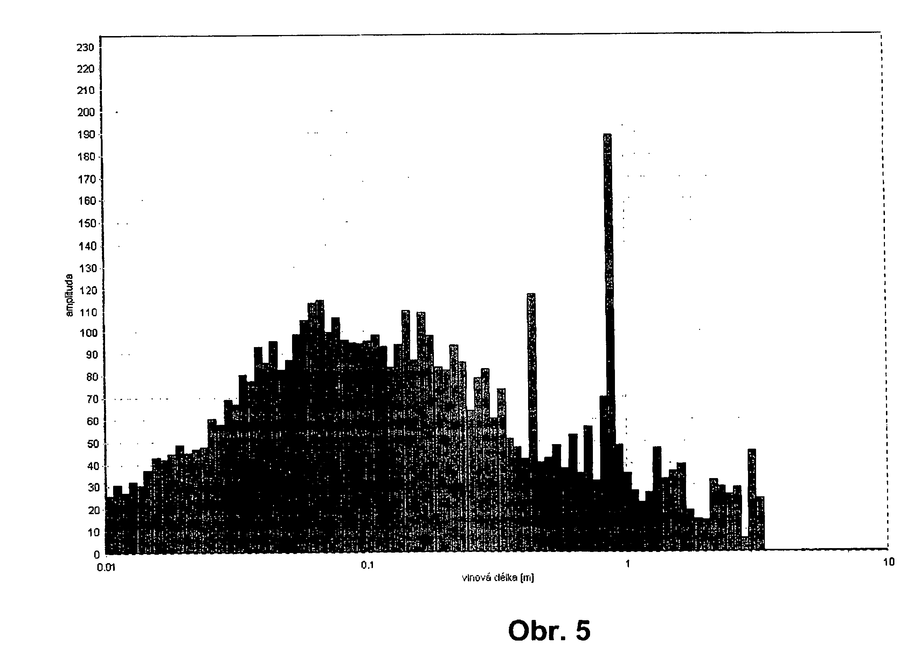

The invention is schematically shown in the drawing where the Fig. 1 shows schematic lay-out of the device for measuring of longitudinal irregularities of textile fibre slivers and of similar longitudinal formations, the Fig. 2 shows an example of block lay-out of electronic section of the device for measuring of longitudinal irregularities of textile fibre slivers and of similar longitudinal formations, the Fig. 3 shows demonstration of percentage variations of length sliver weight, the Fig. 4 shows demonstration of amplitudes of wave components of the sliver, the Fig. 5 shows demonstration of amplitudes of wave components of the sliver and the Fig. 6 shows demonstration of length variation curve of the sliver .

## Examples of embodiment

The method of measuring of longitudinal irregularities of textile fibre slivers and of similar longitudinal formations will be described in examples of embodiment and functioning of the device for measuring of longitudinal irregularities of textile fibre slivers and of similar longitudinal formations.

The measured sliver 1 is directed into the condensing funnel 2, e.g. in the form of a small funnel with a defined diameter and shape of the outlet opening.

The condensing funnel 2 is attached to the sensor 3 that at the movement of the sliver 1 detects the drag force caused by passing the sliver 1 through the condensing funnel 2 at simultaneous condensing the sliver 1 in the condensing funnel 2. This force, at suitably defined diameter of the outlet opening of the condensing funnel 2 corresponds to the length weight of the sliver 1. We can attain the change in the measuring range of the device through a simple replacement of the condensing funnel 2 for another one with the corresponding diameter of the outlet opening.

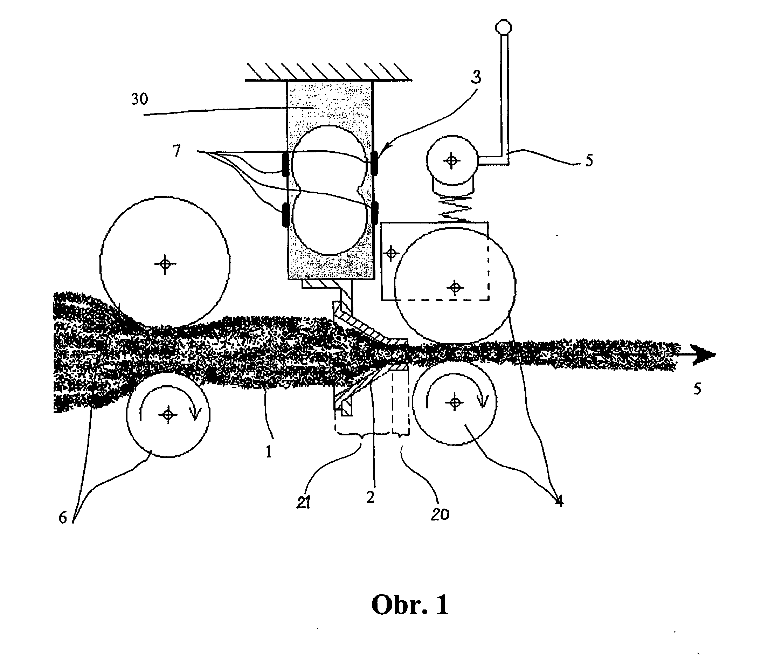

In the shown example of embodiment the sensor 3 is represented by a deformation component 30 with glued-on strain gauges 7 connected with advantage in Wheatstone bridge. The advantage of the sensor 3 with the deformation component 30 according to Fig. 1 is the insensitivity to the position of point of action of the drag force measured.

From the condensing funnel **2** the sliver **1** is drawn off by means of a pair of draw-off rollers **4** ensuring the smooth running of the sliver **1** through the condensing funnel 2.

The device is further equipped with a sensing device of a length of the measured sliver **1.** This sensing device may be created e.g. by an incremental sensor of rotation at least of one of the draw-off rollers 4, e.g. the lower draw-off roller 4, that is motor driven. It can be arranged in such a way that only the upper draw-off roller 4 is driven or even both draw-off rollers 4 may be driven.

The upper draw-off roller 4 is in a defined way pressed through the sliver **1** to the lower draw-off roller 4, in an example shown in Fig. 1 this is realised through the tensioning mechanism 5. The corresponding pressure ensures that there is a proper draw-off of the sliver 1 from the condensing funnel 2. In the not represented example of embodiment, the lower draw-off roller 4 is being pressed to the upper draw-off roller 4.

For the measurement accuracy not to be reduced by further disturbing drag forces before the condensing funnel 2 which could be initiated e.g. by sticking of the sliver **1** at withdrawal of the sliver **1** directly from the transportation can, there is a pair of feeding rollers 6 inserted between the not shown stock of the sliver **1** and the condensing funnel **2.** At least one of them, e.g. the lower feeding roller 6 is motor driven and the second of them, e.g. the upper feeding roller **6** presses the sliver **1** to the lower feeding roller **6** with the pressure mechanism which is not shown. The peripheral speed of feeding rollers 6 is slightly higher than the peripheral speed of draw-off rollers 4. In the example of execution which is not shown, the lower feeding roller 6 is pressed to the upper feeding roller 6.

The condensing funnel has a tapered section 21 which is in the movement direction **5** of the sliver **1** finished with the constant section **20** with the outlet opening of the condensing funnel 2. The tapered section 21 of the condensing funnel 2 may have a form of taper or can be otherwise suitably formed. The constant section 20 of the condensing funnel 2 may have a cylindrical form with a circular outlet opening or can be of a non-rolled form e.g. with cross-section and an outlet opening in the form of oval or in other suitable form. The constant section 20 may be of an optional length e.g. from 1 mm to 8 mm. If the length of the constant section 20 is higher, also the value of measured drag force is increasing, but simultaneously the spectral resolution is decreasing because the constant section 20 filters-off the rapid changes of the length weight of the sliver **1**. The shortest length of the constant section **20** may, with advantage, correspond to the wall thickness of the tapered section 21 of the condensing funnel 2, and after then the measured variations in courses of the length weight of the sliver 1 contain even the short-wave spectra with a wave length of e.g. from 1 mm.

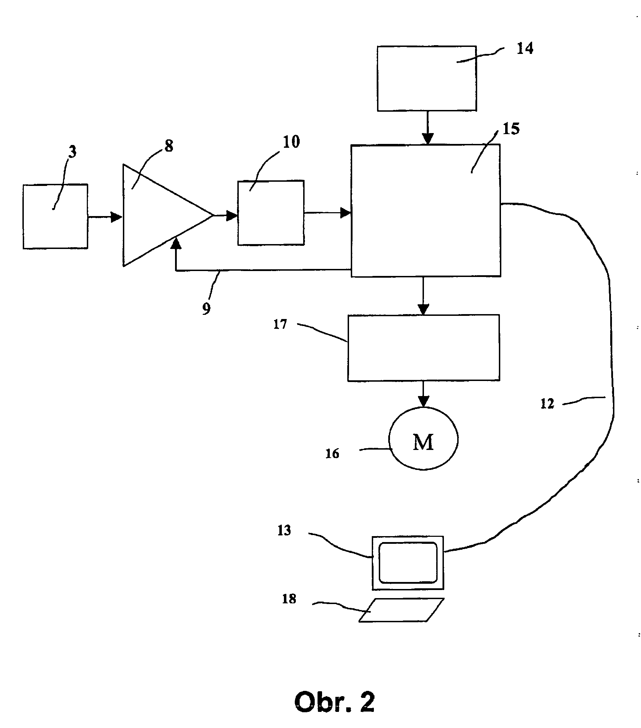

According to the execution shown in the Fig. 2 the signal of the strain-gauge sensor 3 is amplified by the amplifier 8 which, with advantage, has an adjustable amplifying, e.g. adjustable software amplifying to several levels realised by the circuit 9. Next to this there is the A/D converter 10 included whose (high) resolution enables calibration of zero status of the sensor 3 e.g. software calibration of zero status of the sensor 3. The data digitized in this form are transferred into the programmable gate array 15, to which the sampling signal is brought simultaneously, e.g. from the incremental sensor 14 connected to one of the draw-off rollers **4.** The programmable gate array **15,** which may, in the not shown example, be replaced by a suitable (e.g. customised) integrated circuit or by a suitable microprocessor system etc., processes the data measured while the samples of drag force received represent already the function of the length of measured sliver **1.** The programmable gate array **15,** a suitable integrated circuit or a suitable microprocessor system transfers the data processed through the connection 12 (wire or wireless) to the control computer 13 for further processing. The computer 13 may be connected to the printer 18 for printing of measured and calculated data. The programmable gate array **15,** a suitable integrated circuit or a suitable microprocessor system create the digital processing device.

The programmable gate array 15 or a suitable integrated circuit or a suitable microprocessor system are further coupled with a power member 17, which controls the drive 16 of the draw-off rollers 4.

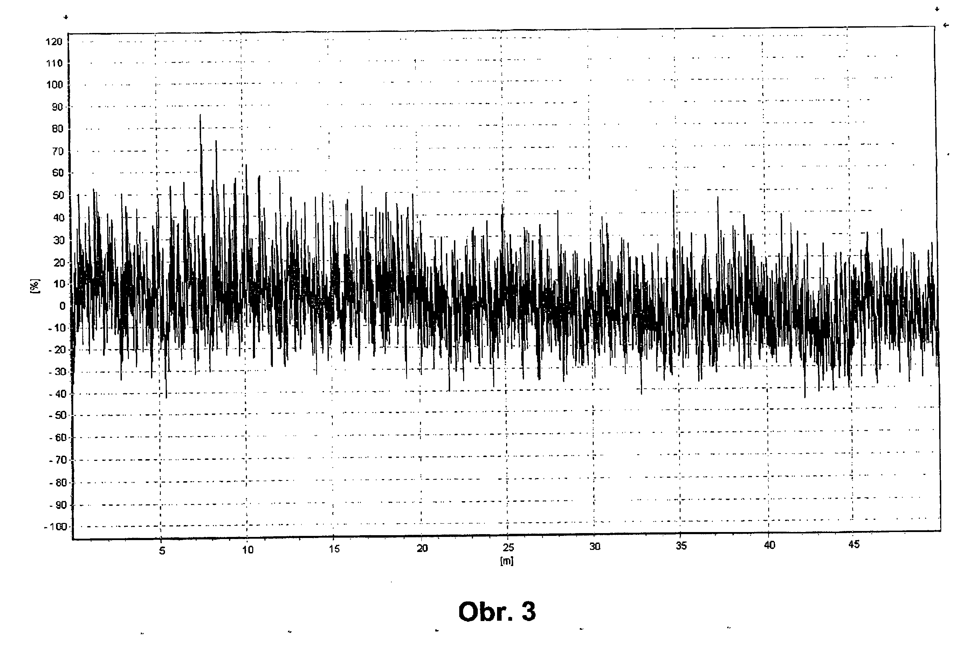

If, during measuring of textile slivers 1 , the standard curve for transfer of measured drag force to the length weight of the sliver **1** is used then it is possible, already during the measuring itself, to display the data to the measured sections of the sliver 1, namely either the data on real course of the length weight of the measured sliver **1** expressed in units "ktex" (length weight "tex" = weight of the sliver **1** in grams to 1000 metres of the sliver length **1**) or as a percentage variation of length weight of the sliver 1 received by subtracting the mean values as per the Fig. 3. Next to this, it is possible to determine and display the point and column spectrograms of wave lengths and also the course of the length variation curve CV(L) with the worksheet values CV for the selected wave lengths. These graphs or the values of which they are created may after then be compared with other courses of the respective values. All values, data, graphs and charts may be immediately printed, or the measured data may be exported into the files of appropriate formats for a later processing e.g. using the computer of PC type or for a later visualisation.

For measuring of mass irregularity of the sliver 1 in absolute values of the length weight of the sliver 1 it is possible to calibrate the device through measurement performed on graduated slivers 1 of a known length and weight or through measuring of row of slivers 1 of known length weights in tex units

$$T\,[tex] = \frac{m\,[g]}{l\,[m]} \cdot 10^3$$

An exemplary course of the percentage variations of length weight of the sliver **1** is shown in Fig. 3, at the same time the value of the variation coefficient CV serves for a quick (orientation) assessment of regularity of measuring.

$$CV = \frac{100}{\bar{x}} \sqrt{\frac{1}{T} \int_0^T (x_i - \bar{x})^2\, dt} \quad [\%]$$

CV - variation coefficient
T - sampling period
$x_i$ - instantaneous value
$\bar{x}$ - mean value

Thanks to the computer it is possible to perform various operations on the created graphs, e.g. it is possible to enlarge the selected portions of graphs, the values may be better read off from the graph, the coordinates of cursors towards the symmetry axes may be visualised in a convenient manner, the absolute and local maxima and minima of the graph may be searched automatically, etc.

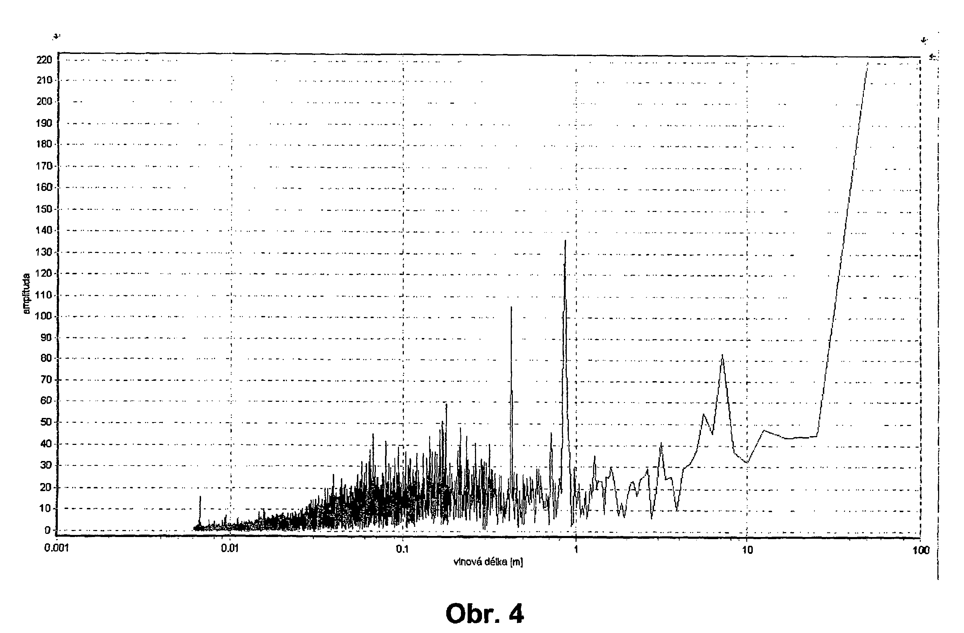

The Fig. 4 shows an example of a graph for amplitudes of wave components of the sliver 1. The calculation preferably uses so called "quick Fourier transformation" (FFT) when the function of time is superseded by the function of length. The advantage of this calculation is that it enables a detailed displaying of particular disturbing wave lengths, on basis of which the source of periodical disturbances of the sliver **1** may be exactly determined e.g. the disturbances caused by the motional parts of production machines, etc. In the textile practice the column wave spectra with a constant percentage bandwidth according to the Fig . 5 are spread.

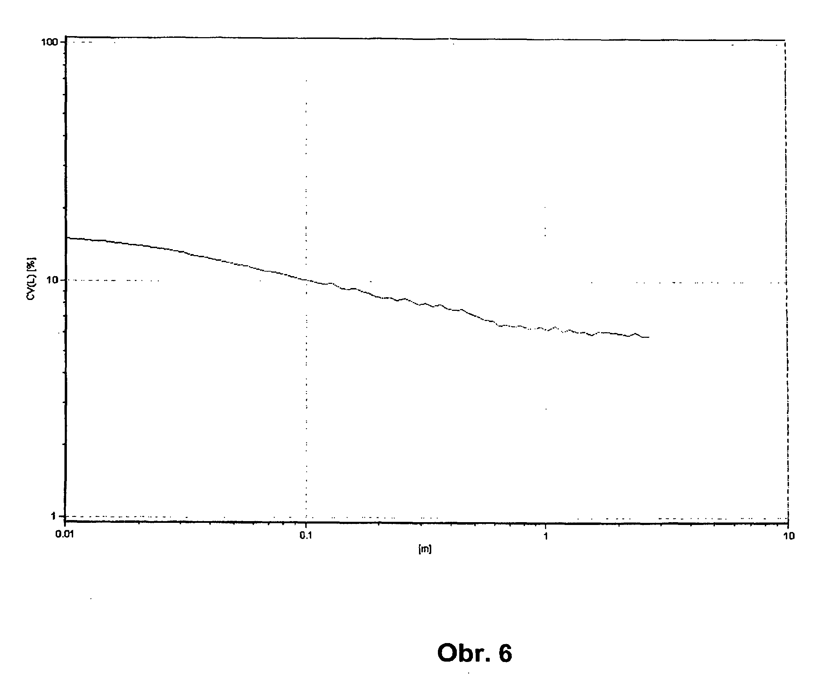

The Fig. 6 shows an example of the length variation curve CV(L), that represents the basic criterion for evaluation of quality of the spinning slivers **1**. The quality of the sliver **1** is characterized by the slope of a curve, its shape and the CV values for the particular wave lengths. For an easier demonstration, the CV values for the selected wave lengths are illustrated in a chart next to the graph, on the right hand side. When printing the graph according to the Fig. 6, this chart of the CV values for the selected wave lengths is being printed out automatically

The CV(L) curve is being, with advantage, created up to the wave length corresponding to the one twentieth of the measured length of the sliver **1.** It is because the determined values would not be statistically sufficiently evidential for the longer wave lengths as these wave lengths do not have a sufficient number of periods in the measured section.

Relations for determination of the length variation curve CV(L) are as follows:

$$\overline{m}(L) = \frac{1}{k}\sum_{i=1}^{k} m_i(L), \quad \sigma(L) = \sqrt{\frac{1}{k-1}\sum_{i=1}^{k}(m_i(L) - \overline{m}(L))^2} \;, \quad CV(L) = \frac{\sigma(L)}{\overline{m}(L)} * 100 \;\; [\%]$$

where:

$\overline{m}$ - mean weight of sections of the sliver having the L length

$\sigma(L)$ - mean quadratic deviation of sections of the sliver having the L length

$CV(L)$- variation coefficient of sections of the sliver having the L length

$CV(L)$L = 0.05, 0.1, 0.2, 0.3, ..... 10 ..... 100 m

k - total number of measuring sections if the measured sliver is divided into the sections having the L length

The measuring device may be modified as to the software, e.g. by the new evaluation methods, etc. through which it can be quite easily and continuously upgraded.

### Industrial applicability

The invention is applicable first of all in the textile production.

### Claims

**1.** Method of measuring the longitudinal irregularity of sliver of textile fibres and of a similar longitudinal formation, **characterized by** that the sliver (1) or a similar longitudinal formation passes through the condensing funnel during the measuring, at the same time the length of the sliver (1) or of a similar longitudinal formation passed through the condensing funnel (2) is being measured and the value of forces at which the sliver (1) or a similar longitudinal formation is acting on the condensing funnel (2) and based on the course of forces acting on the condensing funnel (2) the parameters of mass irregularity of sliver (1) or of a similar longitudinal formation are determined.

**2.** A method as claimed in the claim 1, **characterized by** that, the rapid changes of length weight of the sliver (1) or of a similar longitudinal formation are filtered off during measuring.

**3.** A device for measuring the mass irregularity of sliver of textile fibres and of a similar longitudinal formation containing the feeding, draw-off and measuring mechanism of sliver of textile fibres and of a similar longitudinal formation **characterized by** that the measuring mechanism contains the condensing funnel of the sliver (1) and of a similar longitudinal formation which is positioned on the sensor (3) that is coupled with the device for processing the signal from the sensor (3), at the same time this is coupled with sensing device of length of sliver (1) and of a similar longitudinal formation.

**4.** A device as claimed in the claim 3, **characterized by** that, the condensing funnel has a tapered section (21), which passes to the constant section (20) with an outlet opening of the condensing funnel (2).

**5.** A device as claimed in any of the claims 3 or 4, **characterized by** that the condensing funnel is positioned in a removable way on the sensor (3).

**6.** A device as claimed in any of the claims 3 to 5, **characterized by** that the sensor (3) is created by the deformation component (30) with glued-on strain gauges (7) connected in the Wheatstone bridge.

**7.** A device as claimed in any of the claims 3 to 6, **characterized by** that the sensing device of length of the sliver (1) and of a similar longitudinal formation contains at least one sensor of at least of one mechanism from the pair of feeding mechanism of the sliver (1) and of a similar longitudinal formation — draw-off mechanism of the sliver (1) and of a similar longitudinal formation.

**8.** A device as claimed in the claim 7, **characterized by** that the feeding and/or draw-off mechanism of the sliver (1) contains a pair of one to another pressing rollers (6, 4) out of which at least one is coupled with the drive .

**9.** A device as claimed in any of the claims 3 to 8, **characterized by** that the equipment for processing the signal from the sensor (3) contains on its inlet from the sensor (3) the amplifier (8), after which the A/D converter (10) is positioned and after which the digital processing equipment is included, into which the sampling signal is brought simultaneously, at the same time the digital processing equipment can be interconnected with the controlling computer (13) through connection(12).

**10.** A device as claimed in the claim 9, **characterized by** that the digital processing equipment is coupled with the power member (17) of drive control (16) of the draw-off mechanism.

**Obr. 1**

**Obr. 2**

**Obr. 3**

Obr. 4

Obr. 5

**Obr. 6**